# EUROPEAN PATENT APPLICATION

(11) **EP 2 741 223 A1**
(43) Date of publication of application: **11.06.2014**
(21) Application number: 13193503.3
(22) Date of filing: 19.11.2013
(51) Int. Cl.: G06F 19/00

(54) **Use of neutral loss mass to reconstruct MS-2 spectra in all-ions fragmentation**

(30) Priority: 20.11.2012 US 201213682384
(71) Applicant: Thermo Finnigan LLC, San Jose, CA 95134 (US)
(72) Inventor: Wright, David A, Livermore, CA California 94550 (US)
(74) Representative: Boult Wade Tennant

(57) **Abstract**

A method is provided for acquiring and interpreting data using a mass spectrometer, said method comprising: (a) generating a multiplexed mass spectrum using the mass spectrometer system, the multiplexed mass spectrum comprising a superposition of a plurality of product-ion mass spectra comprising a plurality of product-ion types having respective product-ion mass-to-charge (*m*/*z*) ratios, each product-ion mass spectrum corresponding to fragmentation of a respective precursor-ion type formed by ionization of a chemical compound, each precursor-ion type having a respective precursor-ion mass-to-charge (*m*/*z*) ratio; and (b) recognizing a set comprising a precursor-ion type and one or more product-ion types corresponding to each of one or more of the product-ion mass spectra by recognizing one or more losses of a respective valid neutral molecule from each said precursor-ion type.

## Description

### FIELD OF THE INVENTION

This invention relates to methods of analyzing data obtained from instrumental analysis techniques used in analytical chemistry and, in particular, to methods of automatically identifying matches between precursor and fragment ions, without input from or intervention of a user, in all-ions tandem mass spectral data generated in LC/MS/MS analyses that do not include a precursor ion selection step.

### BACKGROUND OF THE INVENTION

Mass spectrometry (MS) is an analytical technique to filter, detect, identify and/or measure compounds by the mass-to-charge ratios of ions formed from the compounds. The quantity of mass-to-charge ratio is commonly denoted by the symbol "*m*/*z*" in which "*m*" is ionic mass in units of Daltons and "*z*" is ionic charge in units of elementary charge, e. Thus, mass-to-charge ratios are appropriately measured in units of "Da/e". Mass spectrometry techniques generally include (1) ionization of compounds and optional fragmentation of the resulting ions so as to form fragment ions; and (2) detection and analysis of the mass-to-charge ratios of the ions and/or fragment ions and calculation of corresponding ionic masses. The compound may be ionized and detected by any suitable means. A "mass spectrometer" generally includes an ionizer and an ion detector.

One can often enhance the resolution of the MS technique by employing "tandem mass spectrometry" (sometimes abbreviated as "MS/MS", MS-2 or MS²), for example via use of a triple quadrupole mass spectrometer. In this technique, a first, or parent, or precursor, ion generated from a molecule of interest can be filtered or isolated in an MS instrument, and these precursor ions subsequently fragmented to yield one or more second, or product, or fragment, ions that are then analyzed in a second MS stage. By careful selection of precursor ions, only ions generated from certain selected analytes are passed to the fragmentation chamber or other reaction cell, such as a collision cell where collision of ions with atoms of an inert gas produces the fragment ions. Because both the precursor and fragment ions are produced in a reproducible fashion under a given set of ionization/fragmentation conditions, the MS/MS technique can provide an extremely powerful analytical tool. For example, the combination of precursor ion selection and subsequent fragmentation and analysis can be used to eliminate interfering substances, and can be particularly useful in complex samples, such as biological samples. Selective reaction monitoring (SRM) is one commonly employed tandem mass spectrometry technique.

The hybrid technique of liquid chromatography-mass spectrometry (LC/MS) is an extremely useful technique for detection, identification and (or) quantification of components of mixtures or of analytes within mixtures. This technique generally provides data in the form of a mass chromatogram, in which detected ion intensity (a measure of the number of detected ions) as measured by a mass spectrometer is given as a function of time. In the LC/MS technique, various separated chemical constituents elute from a chromatographic column as a function of time. As these constituents come off the column, they are submitted for mass analysis by a mass spectrometer. The mass spectrometer accordingly generates, in real time, detected relative ion abundance data for ions produced from each eluting analyte, in turn. Thus, such data is inherently three-dimensional, comprising the two independent variables of time and mass (more specifically, a mass-related variable, such as mass-to-charge ratio) and a measured dependent variable relating to ion abundance.

Generally, "liquid chromatography" (LC) means a process of selective retention of one or more components of a fluid solution as the fluid uniformly percolates through a column of a finely divided substance, or through capillary passageways. The retention results from the distribution of the components of the mixture between one or more stationary phases and the bulk fluid, (i.e., mobile phase), as this fluid moves relative to the stationary phase(s). "Liquid chromatography" includes, without limitation, reverse phase liquid chromatography (RPLC), hydrophilic interaction liquid chromatography (HILIC), high performance liquid chromatography (HPLC), normal-phase high performance liquid chromatography (NP-HPLC), ultra high performance liquid chromatography (UHPLC), supercritical fluid chromatography (SFC) and ion chromatography.

Recent improvements in liquid chromatography (LC) throughput and mass spectrometry (MS) detection capabilities have led to a surge in the use of LC/MS-based techniques for screening, confirmation and quantification of ultra-trace levels of analytes. The triple quadrupole mass spectrometer has historically been considered the gold standard for quantitation, and SRM techniques are typically used, for example, for the validation of potential biomarkers. Liquid chromatography-triple quadrupole tandem MS (LC/MS/MS) enables highly selective and sensitive quantification and confirmation of hundreds of target compounds in a single run. Unfortunately, such an approach requires extensive compound-dependent parameter optimization and thus requires MS/MS methods to be developed for each analyte. Consequently, the LC/MS/MS approach is restricted to a limited number of compounds per analysis. Moreover, this approach cannot be used to screen for untargeted chemical constituents and does not allow for post acquisition re-interrogation of data.

Because of the above-noted limitations of triple-quadrupole instruments, there is currently a trend towards full-scan MS experiments in residue analysis. Such full-scan approaches utilize high performance time-of-flight (TOF) or electrostatic trap (such as Orbitrap®-type) mass spectrometers coupled to UHPLC columns and can facilitate rapid and sensitive screening and detection of analytes. The superior resolving power of the Orbitrap® mass spectrometer (up to 100,000 FWHM) compared to TOF instruments (10,000-20,000) ensures the high mass accuracy required for complex sample analysis.

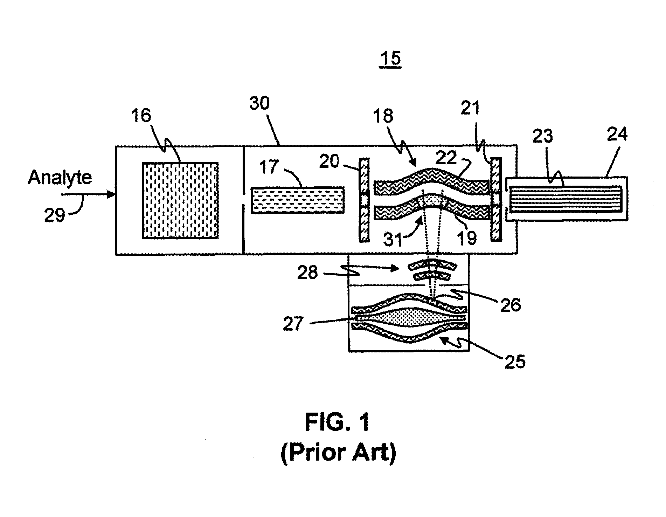

An example of a mass spectrometer system **15** comprising an electrostatic trap mass analyzer such as an Orbitrap® mass analyzer **25** is shown in FIG. 1. Analyte material **29** is provided to a pulsed or continuous ion source **16** so as to generate ions. Ion source **16** could be a MALDI source, an electrospray source or any other type of ion source. In addition, multiple ion sources may be used. The illustrated system comprises a curved quadrupole trap **18** (also known as a "C-trap") with a slot **31** in the inner electrode **19.** Ions are transferred from the ion source **16** to the curved quadrupole trap **18** by ion optics assembly **17** (e.g. an RF multipole). Prior to ion injection, ions may be squeezed along the axis of the curved quadrupole trap **18** by raising voltages on end electrodes **20** and **21.** For ion injection into the Orbitrap® mass analyzer **25,** the RF voltage on the curved quadrupole trap **18** may be switched off, as is well known. Pulses are applied to electrodes **19** and **22** and to an electrode of curved ion optics **28** so that the transverse electric field accelerates ions into the curved ion optics **28.** The converging ion beam that results enters the Orbitrap® mass analyzer **25** through injection slot **26.** The ion beam is squeezed towards the axis by an increasing voltage on a central electrode **27.** Due to temporal and spatial focusing at the injection slot **26,** ions start coherent axial oscillations. These oscillations produce image currents that are amplified and processed. Further details of the electrostatic trap apparatus **25** are described in International Application Publication WO 02/078046, US Pat. No. 5,886,346, US Pat. No. 6,872,938. The ion optics assembly **17,** curved quadrupole trap **18** and associated ion optics are enclosed in a housing **30** which is evacuated in operation of the system.

The system **15** (FIG. 1) further comprises reaction cell **23,** which may comprise a collision cell (such as an octopole) that is enclosed in a gas tight shroud **24** and that is aligned to the curved quadrupole trap **141.** The reaction cell **23,** when used as a collision cell, may be supplied with an RF voltage of which the DC offset can be varied. A collision gas line (not shown) may be attached and the cell is pressurized with nitrogen (or any) gas.

Higher energy collisions (HCD) may take place in the system **15** as follows: Ions are transferred to the curved quadrupole trap **18.** The curved quadrupole trap is held at ground potential. For HCD, ions are emitted from the curved quadrupole trap **18** to the octopole of the reaction cell **23** by setting a voltage on a trap lens. Ions collide with the gas in the reaction cell **23** at an experimentally variable energy which may be represented as a relative energy depending on the ion mass, charge, and also the nature of the collision gas (i.e., a normalized collision energy). Thereafter, the fragment ions are transferred from the reaction cell back to the curved quadrupole trap by raising the potential of the octopole. A short time delay (for instance 30 ms) is used to ensure that all of the ions are transferred. In the final step, ions are ejected from the curved quadrupole trap **18** into the Orbitrap® analyzer **25** as described previously.

The mass spectrometer system **15** illustrated in FIG. 1 lacks a mass filtering step and, instead, causes fragmentation of all precursor ions at once, without first selecting particular precursor ions to fragment. Accordingly, the equivalent of a tandem mass spectrometry experiment is performed as follows: (a) a first sample of ions (comprising a plurality of types of ions) produced from an eluting chemical compound are transferred to and captured by the curved quadrupole trap **18;** (b) the first sample of ions is transferred to the Orbitrap® analyzer **25** as described above for analysis, thereby producing a "full-scan" of the ions; (c) after the first sample of ions has been emptied from the curved quadrupole trap **18,** a second sample of ions from the same chemical compound are transferred through the curved quadrupole trap **18** to the reaction cell **23;** (d) in the reaction cell, a plurality of different types of fragment ions are formed from each of the plurality of ion types of the second sample of the chemical compound; (e) once the Orbitrap® analyzer **25** has been purged of the first sample of ions, the fragment ions are transferred back quadrupole trap **18** and then to the Orbitrap® analyzer **25** for analysis as described above. Such "all-ions-fragmentation scanning" provides a potential multiplexing advantage, but only if the analysis firmware or software can successfully extract precursor-product relationships between the thousands of ions generated in the all-ions-fragmentation scan and the additional thousands of ions present in the full-MS precursor scan.

The spectrometer system **15** illustrated in FIG. 1 is merely a single example of a mass spectrometer system in accordance with the present teachings, or in conjunction with which methods in accordance with the present teachings may be employed. The present teachings may also be employed in conjunction with other mass spectrometer systems having sufficiently high mass precision and resolution - such as time-of-flight (TOF) and other mass spectrometer systems -if those systems are used for all-ions-fragmentation experiments.

Prior approaches to extract precursor-product relationships from all-ions-fragmentation (AIF) data correlating XIC (extracted ion chromatogram) lineshapes among precursor and product scans. Such an approach has been described, for instance in international PCT application Publication No. WO2005/113830 A2 and in U.S. Patent Application Publication No. 2012/0158318 A1, the latter of which is assigned to the assignee of the present invention. Using this correlation technique, reconstructed MS-2 spectra can be produced that include many, if not all, of the ions one would expect from a direct measurement of the MS-2 spectrum of a selected precursor ion.

For those instruments that have rapid cycle times and that can generate many (7-9 or more) full MS and all-ions-fragmentation scans in the few seconds of an expected chromatographic peak, lineshape correlation of the XIC data is a more-important, with regard to producing accurate reconstructed MS-2 spectra, than is high mass accuracy. For example, with enough data points across a chromatographic peak, quality MS-2 spectra can be obtained with only 300 ppm mass accuracy data. If the chromatography is very poor, however, or the instrument is not capable of recording 7-9 or more data points of each scan type across a chromatographic peak, then the XIC correlation method does not work well. The notion of "poor" chromatography refers, in this instance, to incomplete separation of compounds such that the elution profiles of two or more compounds strongly overlap in time.

### SUMMARY

The inventor of the present invention has realized that, for AIF experimental data in which the chromatographic separation is poor or very poor, or in which the instrument is not capable of recording seven or more data points of each scan type across a peak, then precise values of the ion *m*/*z* values can be used instead of profile correlations in order to match precursor ions with their correct product or fragment ions. The inventor has realized that one can exploit the fact that an instrument tends to have higher precision than accuracy by calculating masses of neutral molecules lost during ion fragmentation or reaction (neutral loss values) rather than the actually measured *m*/*z* values of ions because the neutral loss values comprise mass differences. If only a minimal amount of chromatographic separation is available with regard to co-eluting compounds, then it is advantageous to filter the AIF data by considering only those product ions whose neutral loss from a proposed precursor ion corresponds to a valid and probabilistically likely chemical formula.

The novel techniques described herein can be used in conjunction with a mass spectrometer system (such as the system **15** illustrated in FIG. 1) that interleaves precursor and product scans so as to decompose overlapping MS-2 spectra in product-ion scans and statistically relate each such resolved spectrum back to a precursor mass in a precursor-ion scan. Clearly, the provision of high mass precision is desirable in order to limit the number of such ions. It is preferable that the mass spectra have a precision of on the order of 5 parts-per-million (ppm) or better. Such mass precision is available on commercially available electrostatic trap mass spectrometer systems (e.g., Orbitrap® mass spectrometer systems), on time-of-flight (TOF) mass spectrometer systems, as well as others.

It has been found that execution of just the steps described above is very effective and often leads to correct synthetic MS/MS spectra without the necessity of additional analysis. That *m*/*z* values that are determined gain credibility through their correspondence to plausible chemical formulae. And, since mass spectrometers such as those described herein typically have better precision than accuracy, the criterion used is that the neutral loss mass should correspond to a chemical formula of a neutral molecule, not the precursor or fragment masses.

According to first aspect of the invention, a method is provided for acquiring and interpreting data using a mass spectrometer, said method comprising: (a) generating a multiplexed mass spectrum using the mass spectrometer system, the multiplexed mass spectrum comprising a superposition of a plurality of product-ion mass spectra comprising a plurality of product-ion types having respective product-ion mass-to-charge (*m*/*z*) ratios, each product-ion mass spectrum corresponding to fragmentation of a respective precursor-ion type formed by ionization of a chemical compound, each precursor-ion type having a respective precursor-ion mass-to-charge (*m*/*z*) ratio; and (b) recognizing a set comprising a precursor-ion type and one or more product-ion types corresponding to each of one or more of the product-ion mass spectra by recognizing one or more losses of a respective valid neutral molecule from each said precursor-ion type.

According to some embodiments, the step (a) of generating a multiplexed mass spectrum using the mass spectrometer may comprise generating the spectrum with a precision of 5 parts-per-million or better. According to some embodiments, the step (a) of generating a multiplexed mass spectrum using the mass spectrometer comprises generating the multiplexed mass spectrum as one of a plurality of multiplexed mass spectra generated during sequential introduction into the mass spectrometer system of compounds corresponding to a chromatograph elution peak, wherein the number of multiplexed mass spectra corresponding to the elution peak is fewer than seven.

According to some embodiments, the recognizing of one or more losses of a respective valid neutral molecule from each said precursor-ion type may comprise: (b1) determining the charge state and mass of each said precursor-ion type; (b2) determining the charge state and mass of each of the plurality of product-ion types; (b3) subtracting the mass of each of the plurality of product-ion types from the mass of each said precursor-ion type so as to generate a list of tentative molecular masses for each said precursor-ion type; (b4) tabulating a list of tentative molecular formulas for each tentative molecular mass; (b5) ranking each list of tentative molecular formulas according to chemical likelihood rules and an isotopic pattern correspondence; (b6) assigning the highest-ranked tentative molecular formula to its respective tentative molecular mass if the ranking of the highest-ranked tentative molecular formula exceeds a threshold value; and (b7) for each pair of precursor-ion type and product-ion type corresponding to a tentative molecular mass corresponding to an assigned tentative molecular formula, recognizing the assigned tentative molecular formula as a loss of a valid neutral molecule. Some embodiments may include normalizing the set of intensities of an isotopic distribution pattern of each of the plurality of product-ion types and of the precursor-ion type to the intensity of a monoisotopic mass of each respective product-ion type and precursor-ion type and subtracting the normalized isotopic distribution pattern of each of the plurality of product-ion types from the mass of each said precursor-ion type so as to generate a generate a list of molecular isotopic patterns, each corresponding to a respective one of the tentative molecular masses. Some embodiments may include calculating a plurality of likelihood scores for each tentative molecular formula, each likelihood score corresponding to a respective rule and calculating the ranking for each tentative molecular formula as the product of the plurality of the likelihood scores multiplied by an isotopic pattern correlation score.

According to a second aspect of the invention, there is provided an apparatus comprising: (i) a mass spectrometer system; and (ii) a programmable electronic processor electrically coupled to the mass spectrometer, the programmable processor comprising instructions operable to cause the programmable processor to: (a) cause the mass spectrometer system to generate a multiplexed mass spectrum using the mass spectrometer system, the multiplexed mass spectrum comprising a superposition of a plurality of product-ion mass spectra comprising a plurality of product-ion types having respective product-ion mass-to-charge (*m*/*z*) ratios, each product-ion mass spectrum corresponding to fragmentation of a respective precursor-ion type formed by ionization of a chemical compound, each precursor-ion type having a respective precursor-ion mass-to-charge (*m*/*z*) ratio; (b) receive the multiplexed mass spectrum from the mass spectrometer system; and (c) recognize a set comprising a precursor-ion type and one or more product-ion types corresponding to each of one or more of the product-ion mass spectra by recognizing one or more losses of a respective valid neutral molecule from each said precursor-ion type. The apparatus may further comprise (iii) a chromatograph for providing a stream of separated chemical substances to the mass spectrometer system, wherein the multiplexed mass spectrum comprises one of a plurality of multiplexed mass spectra generated during sequential introduction into the mass spectrometer system of the separated chemical substances corresponding to a chromatograph elution peak, wherein the number of multiplexed mass spectra corresponding to the elution peak is fewer than seven. In many embodiments, the mass spectrometer system comprises an analytical precision of 5 parts-per-million or better. In some embodiments, the mass spectrometer system may include a time-of-flight (TOF) mass analyzer. In various embodiments, the mass spectrometer system may include an electrostatic trap mass analyzer.

### BRIEF DESCRIPTION OF THE DRAWINGS

The above noted and various other aspects of the present invention will become apparent from the following description which is given by way of example only and with reference to the accompanying drawings, not drawn to scale, in which:
FIG. 1 is a schematic illustration of an example of a mass spectrometer system comprising an electrostatic trap mass analyzer such as an Orbitrap® mass analyzer;
FIG. 2 is a schematic diagram of a system for generating and automatically analyzing chromatography / mass spectrometry spectra in accordance with the present teachings;
FIG. 3 is a perspective view of a three-dimensional graph of chromatography-mass spectrometry data, in which the variables are time, mass (or mass-to-charge ratio, *m*/*z*) and ion abundance;
FIGS. 4A-4B provide a flowchart of a method for generating automated correlations between all-ions precursor ions and all-ions-fragmentation product ions in accordance with the present teachings;
FIG. 5A is a flowchart of a method for automated spectral peak detection and quantification;
FIG. 5B is a schematic example of decomposing a complexly shaped chromatogram trace into resolved peaks;
FIG. 6A is a mass spectrum of a precursor ion generated from buspirone;
FIG. 6B is a reconstructed MS-2 spectrum of the product ions formed by fragmentation of the buspirone precursor ion having *m*/*z* = 386.2536, wherein the reconstruction is performed in accordance with the present teachings; and
FIG. 6C is a reconstructed MS-2 spectrum of the product ions formed by fragmentation of the buspirone precursor ion having *m*/*z* = 386.2536, wherein the reconstruction is performed by the method of correlating extracted ion chromatogram profiles.

### DETAILED DESCRIPTION

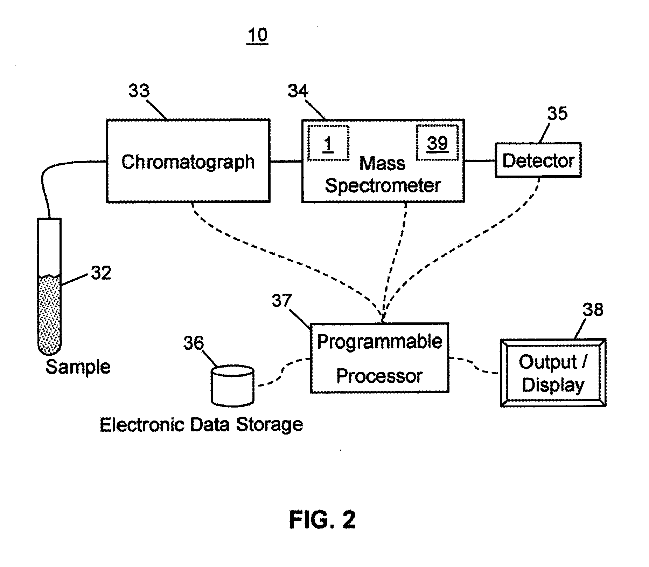

The present invention provides methods and apparatus for correlating precursor and product ions in all-ions fragmentation experiments. The automated methods and apparatus described herein do not require any user input or intervention. The following description is presented to enable any person skilled in the art to make and use the invention, and is provided in the context of a particular application and its requirements. Various modifications to the described embodiments will be readily apparent to those skilled in the art and the generic principles herein may be applied to other embodiments. Thus, the present invention is not intended to be limited to the embodiments and examples shown but is to be accorded the widest possible scope in accordance with the features and principles shown and described. The particular features and advantages of the invention will become more apparent with reference to the appended FIGS. 2-5, taken in conjunction with the following description.

The present disclosure makes use of the terms "ion" (or "ions" in the plural) and "ion type" (or "ion types" in the plural). For purposes of this disclosure, an "ion" is considered to be a single, solitary charged particle, without implied restriction based on chemical composition, mass, charge state, mass-to-charge (*m*/*z*) ratio, etc. A plurality of such charged particles comprises a collection of "ions". An "ion type", as used herein, refers to a category of ions - specifically, those ions having a given monoisotopic *m*/*z* ratio - and, most generally, includes a plurality of charged particles, all having the same monoisotopic *m*/*z* ratio. This usage includes, in the same ion type, those ions for which the only difference or differences are one or more isotopic substitutions. One of ordinary skill in the mass spectrometry arts will readily know how to recognize isotopic distribution patterns and how to relate or convert such distribution patterns to monoisotopic masses. Occasionally, the word "ion" is used herein in adjective form, as in "precursor-ion mass spectrum" or "product-ion mass spectrum". This latter usage should be understood as referring to any number (one or more) of charged particles- but, generally, a large plurality of such charged particles. Thus, the term "precursor-ion mass spectrum" may be generally understood as referring to a mass spectrum of precursor ions. The term "scan" as used herein is used loosely to refer to any mass spectrum - such as a precursor-ion mass spectrum, a product-ion mass spectrum, both a precursor-ion mass spectrum and an associated product-ion mass spectrum considered together, etc. This terminology usage is employed even though many instances of mass spectrometer instruments that may produce data suitable for analysis according to the present teachings are not, strictly speaking, mass-scanning-type instruments. For instance, the mass spectrometer system **15** illustrated in FIG. 1 is not a mass-scanning type of instrument.

### General Considerations

FIG. 2 is a schematic diagram of a system for generating and automatically analyzing chromatography / mass spectrometry spectra in accordance with the present teachings. A chromatograph **33,** such as a liquid chromatograph, high-performance liquid chromatograph or ultra high performance liquid chromatograph receives a sample **32** of an analyte mixture and at least partially separates the analyte mixture into individual chemical components, in accordance with well-known chromatographic principles. As a result, the at least partially separated chemical components are transferred to a mass spectrometer **34** at different respective times for mass analysis. As each chemical component is received by the mass spectrometer, it is ionized by an ionization source **1** of the mass spectrometer. The ionization source **1** may produce a plurality of ions (i.e., a plurality of precursor ions) comprising differing charges or masses from each chemical component. Thus, a plurality of ions of differing mass-to-charge ratios may be produced for each chemical component, each such component eluting from the chromatograph at its own characteristic time. These various ions are analyzed and detected by the mass spectrometer together with its detector **35** and, as a result, appropriately identified according to their various mass-to-charge ratios. As illustrated in FIG. 1, the mass spectrometer comprises a reaction cell **39** to fragment or cause other reactions of the precursor ions but may lack a mass filtering step for selection of particular ions to introduce into the reaction cell. In such a situation, the reaction cell, instead, causes reactions to or fragmentation of all ions at once.

Still referring to FIG. 2, a programmable processor **37** is electronically coupled to the detector of the mass spectrometer and receives the data produced by the detector during chromatographic / mass spectrometric analysis of the sample(s). The programmable processor may comprise a separate stand-alone computer or may simply comprise a circuit board or any other programmable logic device operated by either firmware or software. Optionally, the programmable processor may also be electronically coupled to the chromatograph and/or the mass spectrometer in order to transmit electronic control signals to one or the other of these instruments so as to control their operation. The nature of such control signals may possibly be determined in response to the data transmitted from the detector to the programmable processor or to the analysis of that data. The programmable processor may also be electronically coupled to a display or other output **38,** for direct output of data or data analysis results to a user, or to electronic data storage **36.** The programmable processor shown in FIG. 2 is generally operable to: receive a precursor ion chromatography / mass spectrometry spectrum and a product ion chromatography / mass spectrometry spectrum from the chromatography / mass spectrometry apparatus and to automatically perform the various data analysis, data retrieval and data storage operations in accordance with the various methods discussed below.

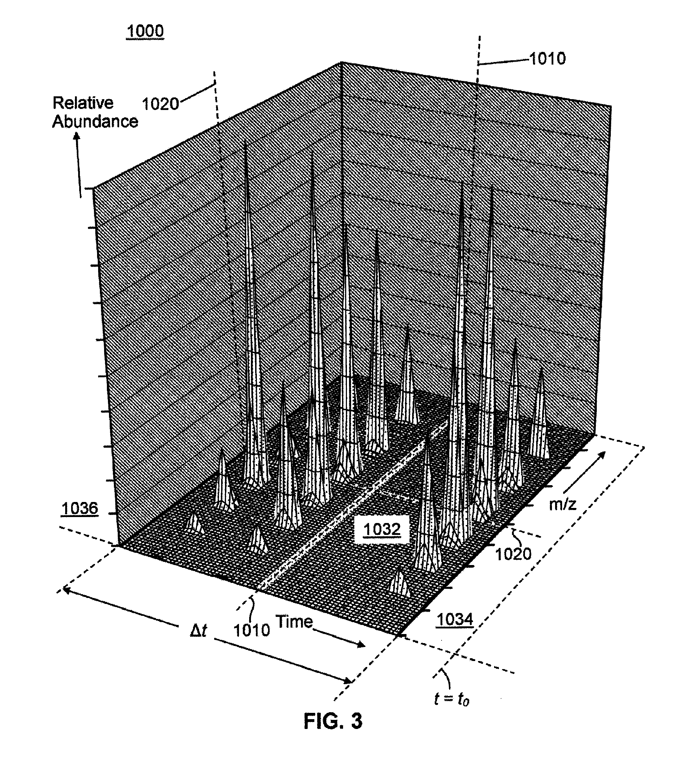

FIG. 3 is a perspective view of a three-dimensional graph of hypothetical LC/MS data. As is common in the representation of such data, the variables time and mass (or mass-to-charge ratio, *m*/*z*) are depicted on the "floor" of the perspective diagram and the variable representing ion abundance (for instance, detected ion current) is plotted in the "vertical" dimension of the graph. Thus, ion abundance is represented as a function of the other two variables, this function comprising a variably shaped surface above the "floor". Each set of peaks dispersed and in line parallel to the *m*/*z* axis represents the various ions produced by the ionization of a single eluting analyte (or, possibly, of fortuitously co-eluting analytes) at a restricted range of time. In a well-designed chromatographic experiment, each analyte of a mixture will elute from the column (thereby to be mass analyzed) within a particular diagnostic time range. Consequently, either a single peak or a line of mass-separated peaks, each such peak representing a particular ion produced by the eluting analyte, is expected at each elution time (or retention time) range.

For clarity, only a very small number of peaks are illustrated in FIG. 3. In practice, data obtained by a chromatography-mass spectrometry experiment may comprise a very large volume of data. A mass spectrometer may generate a complete "scan" over an entire mass range of interest in a matter of tens to hundreds of milliseconds. As a result, up to several hundred complete mass spectra may be generated every second. Further, the various analytes may elute over a time range of several minutes to several tens of minutes, depending on the complexity of the mixture under analysis and the range of retention times represented.

When the chromatography-mass spectrometry experiment and data generation are performed by a mass spectrometer system that performs both all-ion precursor ion scanning and all-ions product ion scanning, the data for each eluate will logically comprise two data subsets , the various scans of which are interspersed with one another in time, and possibly but not necessarily interleaved with one another in a one-to-one fashion (e.g., see FIG. 3). One of these data subsets will contain the data for the precursor ions and the other data subset will contain the data for the product ions.

Returning to the discussion of FIG. 3, the data depicted therein may comprise an entire stored data file representing results of a prior experiment. Alternatively, the data represent a portion of a larger data set in the process of being acquired by an LC/MS instrument. For instance, the data depicted in FIG. 3 may comprise recently collected data held in temporary computer readable memory, such as a memory buffer, and corresponding to an analysis time window, Δ*t*, upon which calculations are being formed while, at the same time, newer data is being collected. Such newer, not-yet-analyzed data is represented, in time and *m*/*z* space, by region **1034** and the data actually being collected is represented by the line *t*=*t*₀*.* Older data which has already been analyzed by methods of the present teachings and which has possibly been stored to a permanent computer readable medium, is represented by region **1036.** With such manner of operation, methods in accordance with the present teachings are carried out in near-real-time on an apparatus used to collect the data or using a processor (such as a computer processor) closely linked to the apparatus used to collect the data.

Operationally, data such as that illustrated in FIG. 3 is collected as separate mass spectra (also referred to herein as "scans"), each mass spectrum ("scan") corresponding to a particular respective time point. Such mass spectra may be envisioned as residing within planes parallel to the plane indicated by the trace lines **1010** in FIG. 3. Once at least a portion of data has been collected, such as the data in region **1032** in FIG. 3, then the information in the data portion may optionally be logically re-organized as extracted ion chromatograms (or, at least portions thereof). Each such XIC may be envisioned as a cross section through the data in a plane parallel to the plane indicated by trace lines **1020** in FIG. 3. Each XIC represents the elution profile, in time, of ions of a particular mass-to-charge range.

### Method

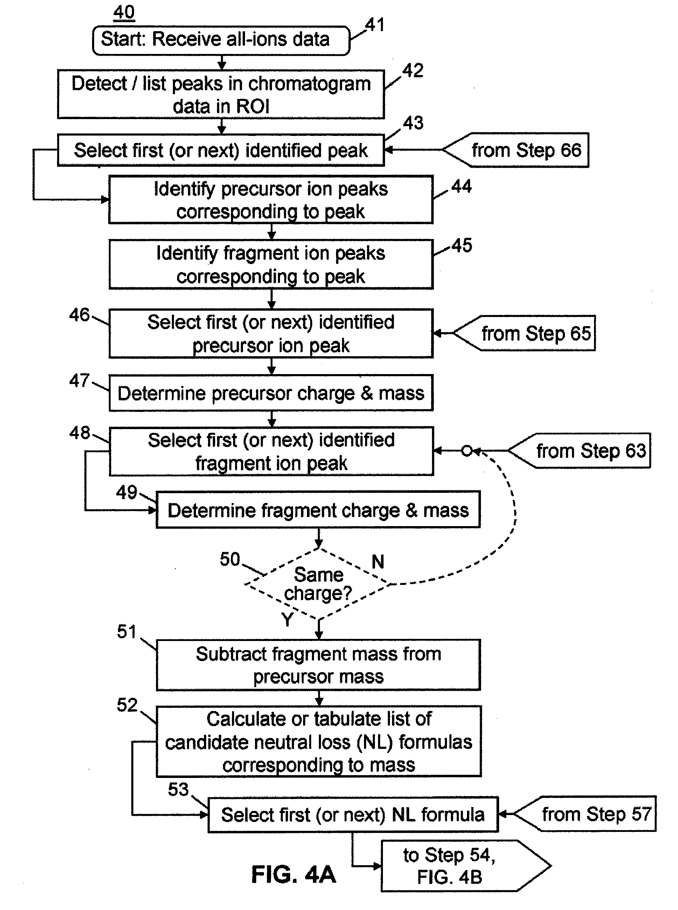

FIGS. 4A-4B present a flowchart of a method **40** for generating automated correlations between all-ions precursor ions and all-ions-fragmentation product ions in accordance with the present teachings. In the initial step, Step **41** (FIG. 4A), all-ions LC/MS/MS data is generated by and received from a chromatograph-mass spectrometer apparatus. Note that the LC/MS data may comprise two data subsets - one data subset containing data for precursor ions and the other data subset containing data for all the fragment ions formed by reaction or fragmentation of all the precursor ions. Each data subset comprises ion abundance (or relative abundance) information as a function of time and *m*/*z.*

The system **15** illustrated in FIG. 1 is capable of repeating the precursor scan and product ion scan sequence five or more times for compounds that elute over a period of 1 second (that is, 10 or more total scans per second). Thus, even though precursor ion and fragment or product ion scans are not exactly coincident in time, the time offset between the acquisition of the precursor ion data and the subsequent product ion data may be considered to be, for purposes of this disclosure, sufficiently small so as to be inconsequential. In those cases in which the chromatic separation and resolution is sufficiently good that the time offset between acquisition of precursor and fragment or product ion data may, in fact, be of consequence, then the XIC correlation methods discussed in the aforementioned U.S. Patent Application Publication 2012/0158318 A1 may be used to advantage.

The calculations of method **40** are performed on a chosen time window of the data set. This time-window corresponds to a current region of interest (ROI) of recently collected data, such as region **1032** of FIG. 3. The region of interest includes data from the precursor ion scan (MS scan) as well as the fragment ion scan (MS/MS scan) and represents a "time slice" or "time window" of the experimental data. In embodiments, this window is 0.6 minutes wide but can vary widely. This time windows represent a small portion of a typical chromatographic experiment which may run for several tens of minutes to on the order of an hour. In some implementations, data dependent instrument control functions may be performed in automated fashion, wherein the results obtained by the methods herein are used to automatically control operation of the instrument at a subsequent time during the same experiment from which the data were collected. For instance, based on the results of the algorithms, a voltage may be automatically adjusted in an ion source or a collision energy (that is applied to ions in order to cause fragmentation) may be adjusted with regard to collision cell operation. Such automatic instrument adjustments may be performed, for instance, so as to optimize the type or number of ions or ion fragments produced.

In Step **42** of the method **40** (FIG. 4A), one or more elution events of compounds within a current region of interest (ROI) are detected. The one or more elution events may be detected as peaks within a mass chromatogram. One non-limiting example of a type of chromatogram which may be employed is a total-ion-current mass chromatogram (TIC), in which total ion current is monitored in "real time" at an ion source. Such data provides a useful representation of the general timing and quantity of elution of compounds from a chromatograph. Another form of chromatogram which may be employed in various implementations is known as a reconstructed total-ion-current chromatogram, in which a chromatographic plot is reconstructed from an array of recorded signal intensities. Still other forms of mass chromatograms which may be employed in certain implementations comprise selected ion monitoring (SIM) mass chromatograms and extracted ion chromatograms (XICs), in which only a subset or limited range of all possible or of all detected *m*/*z* values are considered in the chromatogram. The mass chromatogram may be directly measured and provided by the analytical instrument in real time. The chromatogram provided by the analytical instrument may relate only to detection of precursor ions. Alternatively, a second chromatogram relating to product or fragment ions may also be provided by the analytical instrument. As a still further alternative, the instrument may simply provide raw data in the form of a series of mass spectra, each mass spectrum ("scan") relating to a certain measurement time and comprising intensity data relating to the detection of possibly many different ion masses, such as, for example, precursor ion masses within a certain experimental range of masses. In such cases, the one or more ion chromatograms may be simply calculated in Step **242** by digitally adding together the intensities of the various detected peaks in each scan or by extracting time-varying data in one or more mass ranges (such as extracted ion chromatograms or XICs) by considering variations between multiple individual scans.

The peaks in a total ion chromatogram may be detected by the methods of Parameterless Peak Detection as taught in U.S. Patent No. 7,983,852 assigned to the assignee of the instant invention and incorporated herein in its entirety. In some instances, the region of interest may be defined as a time region around a single detected peak or envelope of peaks - such as, for instance, a time region bounded by limits that are at a distance of twice the standard deviation from a peak maximum on either side of the peak maximum. In some instances, the region of interest may be known or may be estimated prior to performing a particular analysis and may relate to an expected retention time of an expected or target analyte.

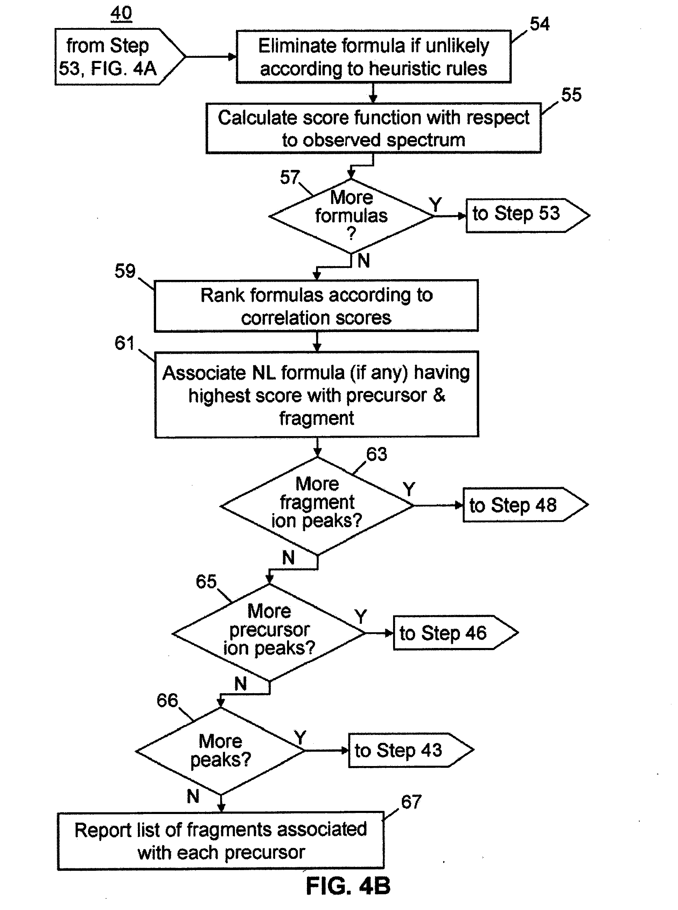

In the subsequent Step **43,** the first such identified peak is selected and subsequently considered in a loop of steps spanning from Step **43** to Step **66** (FIG. 4B). In Steps **44** and **45,** precursor-ion and fragment-ion peaks, respectively, are identified. The precursor-ion and product-ion or fragment-ion peaks may be identified by calculating extracted ion chromatograms as discussed in the aforementioned U.S. Patent Application Publication 2012/0158318 A1, each such ion chromatogram providing a representation of the quantity of ions detected within a respective mass range versus time. Each peak identified in either Step **44** or Step **45** represents a respective mass-to-charge range of ions whose detected intensity rises and falls in correspondence to a particular retention time.

In Step **46** of the method **40,** a first precursor ion peak - as identified in Step **44 -** is selected for consideration within a loop of steps spanning from Step **46** (FIG. 4A) to Step **65** (FIG. 4B). In Step **47,** the charge state and mass of the precursor ion peak under consideration is determined. The charge state may be determined by the spacing between the various peaks of an isotopic distribution of peaks, provided that the instrumental resolution is sufficient. With the magnitude of the charge thus known, the mass of the ion may be thus determined. In Step **48,** a first fragment-ion peak - as identified in Step **45 -** is selected for consideration within a loop of steps spanning from Step **48** (FIG. 4A) to Step **63** (FIG. 4B).

In Step **49,** the charge state and mass of the fragment-ion peak under consideration is determined. The charge state may be determined by the spacing between the various peaks of an isotopic distribution of peaks, provided that the instrumental resolution is sufficient. With the magnitude of the charge thus known, the mass of the ion may be thus determined. Generally, the fragment ion generated by neutral loss should comprise the same charge number as the precursor from which it was formed, the only exceptions being in special cases involving charge transfer. However, assuming collision-induced-dissociation fragmentation not including charge transfer in the dissociation mechanism, then the decision Step **50** is executed. If, in Step **50,** the fragment ion does not comprise the same charge number, then the next identified fragment ion peak is considered (Step **48)** as indicated by the dashed arrow in FIG. 4A. Otherwise, if the two charge numbers are the same, then Step **51** is executed.

In Step **51,** the mass of the fragment ion currently under consideration is subtracted from the mass of the precursor ion currently under consideration so as to provide a tentative mass difference. A list of candidate neutral loss (NL) formulas corresponding to the tentative mass difference is calculated or determined from a table of formula masses in Step **52.** Various databases of molecular formulas and masses are available for this purpose. Subsequently, in Step **53,** the first candidate neutral loss formula is considered. Note that the candidate formulas do not correspond directly to observed masses but, instead, to calculated mass differences between candidate precursor and product ions.

The candidate formula under consideration may, in some embodiments, be eliminated in Step **54** if it is deemed to be unlikely or unrealistic according to various heuristic rules. A list of such rules has been set forth by Kind and Fiehn ("Metabolomic database annotations via query of elemental compositions: Mass accuracy is insufficient even at less than 1 ppm", BMC Bioinformatics 2006, 7:234; "Seven Golden Rules for heuristic filtering of molecular formulas obtained by accurate mass spectrometry", BMC Bioinformatics 2007, 8:105), According to Kind and Fiehn, high mass accuracy (1 ppm or better) and high resolving power are desirable but insufficient for correct molecule identification. With regard to the present teachings, mass precision is a relevant quantity since, according to the methods taught herein, lists of tentative neutral loss molecules are derived by subtracting product-ion masses from precursor-ion masses. With regard to the present teachings, therefore, mass precision of 1 ppm or better is desirable. Such mass precision is available on commercially available electrostatic trap mass spectrometer systems (e.g., Orbitrap® mass spectrometer systems) as well as on time-of-flight (TOF) and other mass spectrometer systems. However, according to Kind and Fiehn, in order to eliminate ambiguities in formula assignments, certain molecules must either be eliminated or determined to be unlikely based on certain rules.

The rules set forth by Kind and Fiehn include a restriction rule relating to the number-of-elements, the LEWIS and SENIOR chemical rules, a rule relating to hydrogen/carbon ratios, a rule relating to the element ratio of nitrogen, oxygen, phosphor, and sulphur versus carbon, a rule relating to element ratio probabilities and a rule relating to the presence of trimethylsilylated compounds. For small organic molecules, such as drugs or their metabolites, the number of elements may be restricted to just the most common elements (e.g., C, H, N, S, O, P, Br and Cl and, possibly Si for some compounds that have been derivitized) and the numbers for nitrogen, phosphor, sulphur, bromine and chlorine should be relatively small relative to carbon. Further, the hydrogen/carbon ratio should not exceed approximately H/C > 3. According to the LEWIS rule, carbon, nitrogen and oxygen are expected to have an "octet" of completely filled s, p-valence shells. The SENIOR rule relates to the required sums of valences.

Some of the Kind and Fiehn rules (for example, valence rules) may be used to positively exclude certain molecules. Others of the rules may be used to calculate likelihoods or probabilities of occurrences based on tabulated observations of large collections of molecular formulas. For example, Kind and Fiehn (2007) present a histogram of hydrogen/carbon ratios for 42,000 diverse organic molecules which may be approximated by a probability density function. Probability density functions - either symmetric or skewed - may be similarly generated with regard to other element ratios. A candidate molecular formula may thus be compared against the various probability functions resulting from application of several of the heuristic rules and assigned a respective likelihood score based on each such rule. As further set forth by Kind and Fiehn, likelihood score may also be calculated in terms of the degree of matching or correlation between theoretical and observed isotopic patterns. In the present case, there is no directly observable isotopic pattern, because the candidate molecules all represent possible losses of neutral molecules. However, a pattern may be generated indirectly by conducting additional operations, in Step **51,** of normalizing the intensities of the observed isotopic distribution patterns of both candidate precursor and product molecules to their respective monoisotopic masses, shifting the mass axes such that monoisotopic masses overlap and then performing a simple spectral subtraction. An isotopic match score may be calculated based on a measure of correlation between the molecular isotopic pattern so calculated and an expected isotopic pattern of a candidate molecular formula.

A respective value of a formula score function is calculated in Step **55,** for those formulas that are not eliminated in Step **54.** In some embodiments, the overall formula score function may be calculated as a product of the individual likelihood scores or correlation scores calculated by application of the individual likelihood rules discussed above. The formulas which are positively excluded by certain of the rules may be eliminated from consideration in Step **54,** prior to this calculation. Alternatively, such excluded formulas may be presumed to comprise scores which are calculated including at least one factor which is equal to zero. In some embodiments, most of the rules may be formulated so as to yield a simple binary "yes" or "no" answer regarding the exclusion of or possible allowance of a certain formula. The final likelihood score for formulas which are not excluded in this fashion may be then calculated from the isotopic correlation scores.

Then, in the loop termination step, Step **57** (FIG. 4B), if there are additional candidate neutral loss formulas to be considered, execution of the method **40** returns to Step **53** and the next candidate neutral loss formula in the list is considered, in turn. Once the value of the formula score function has been calculated for all candidate neutral loss formulas, the various formulas are ranked according to their scores in Step **59.**

In Step **61,** the candidate neutral loss formula (if any) having the highest score may be associated with the precursor ion and fragment ion currently under consideration. However, if there are no candidate neutral loss formulas whose scores are at or above a pre-determined threshold, then no such formula is associated with the precursor ion and fragment ion. The assignment of a neutral loss formula to a precursor-product pair indicates that there is a significant probability that the fragment ion under consideration is related to the precursor ion under consideration by fragmentation of the precursor such that a neutral molecule having the assigned formula is released at the time of formation of the fragment ion.

In the loop termination step, Step **63,** if there are additional fragment-ion peaks within the ROI that have not been considered in conjunction with the precursor ion currently under consideration, then execution of the method **40** returns to Step **48** (FIG. 4A) and the next identified fragment-ion peak is considered, in turn. Otherwise, execution proceeds to the next loop termination step, Step **65.** If, in Step **65,** there are additional precursor-ion peaks within the ROI that have not been considered, then execution of the method **40** returns to Step **46** (FIG. 4A) and the next identified precursor-ion peak is considered, in turn. Otherwise, execution proceeds to the next loop termination step, Step **66.** If, in Step **66,** there are additional chromatogram peaks or elution events that have not been considered, then execution of the method **40** returns to Step **43** (FIG. 4A) and the next identified elution event or peak in the chromatogram is considered, in turn. Otherwise, execution proceeds to the final step, Step **67,** of the method, in which a list of related precursor-fragment pairs, as determined by the values of the formula score function, is reported or stored.

The results are reported to a user (or stored for later use) in Step **67.** The results may include calculated product/precursor matches, information regarding detected peaks or other information. The reporting may be performed in numerous alternative ways-for instance via a visual display terminal, a paper printout, or, indirectly, by outputting the parameter information to a database on a storage medium for later retrieval by a user. The reporting step may include reporting either textual or graphical information, or both. Reported peak parameters may be either those parameters calculated during the peak detection step or quantities calculated from those parameters and may include, for each of one or more peaks, location of peak centroid, location of point of maximum intensity, peak half-width, peak skew, peak maximum intensity, area under the peak, etc. Other parameters related to signal to noise ratio, statistical confidence in the results, goodness of fit, etc. may also be reported in Step **67.**

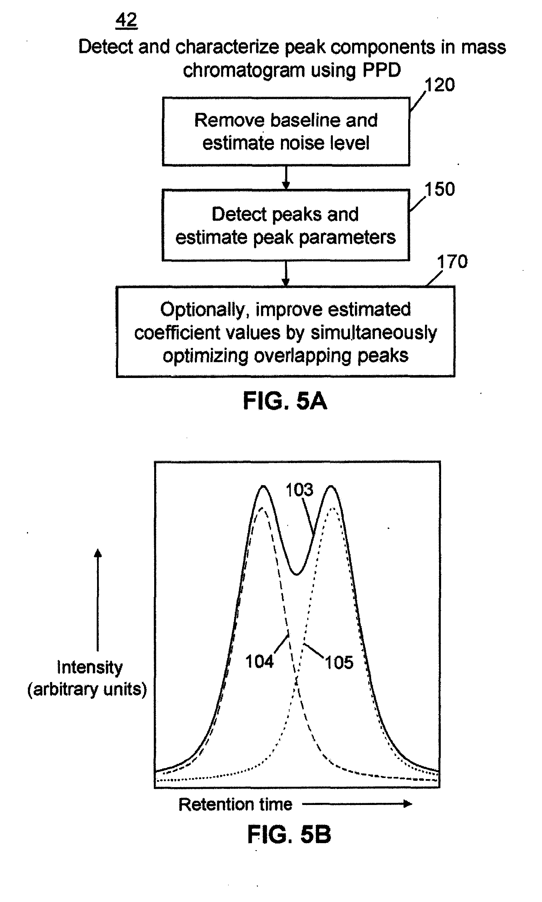

The Step **42** is outlined in brief in FIG. 5A. The individual steps shown in FIG. 5A are discussed in much greater detail in the aforementioned United States Patent number 7,983,852. The Step **42** includes detecting and locating peaks in a chromatogram and may itself be regarded as a particular method, which is shown in flowchart form in FIG. 5A. The purpose of the method **42,** as shown in FIG. 5A, is to decompose a chromatogram trace into component peaks, such as the peaks **104** and **105** schematically illustrated in FIG. 5B. Since the chromatogram includes only the single independent variable of time (e.g., Retention Time). The method **42** is thus directed to detection of peaks in data that includes only one independent variable. The various sub-procedures in the method **42** may be grouped into three basic stages of data processing, each stage possibly comprising several steps as illustrated in FIG. 5A. The first step, Step **120,** of the method **42** is a preprocessing stage in which baseline features may be removed from the received chromatogram and in which a level of random "noise" of the chromatogram may be estimated. The next Step **150** is the generation of an initial estimate of the parameters of synthetic peaks, each of which models a positive spectral feature of the baseline corrected chromatogram. Such parameters may relate, for instance, to peak center, width, skew and area of modeled peaks, either in preliminary or intermediate form.

The final optional Step **170** of the method **42** includes refinement of fit parameters of synthetic peaks determined in the preceding Step **150** in order to improve the fit of the peaks, taken as a set, to the baseline corrected chromatogram. The need for such refinement may depend on the degree of complexity or accuracy employed in the execution of modeling in Step **150.** The Step **170** comprises refinement of the initial parameter estimates for multiple detected chromatographic peaks. Refinement comprises exploring the space of *N* parameters (the total number of parameters across all peaks, i.e. 4 for each Gamma/EMG and 3 for each Gaussian) to find the set of values that minimizes the sum of squared differences between the observed and model chromatogram. Preferably, the squared difference may be calculated with respect to the portion of the chromatogram comprising multiple or overlapped peaks. It may also be calculated with respect to the entire chromatogram. The model chromatogram is calculated by summing the contribution of all peaks estimated in the previous stage. The overall complexity of the refinement can be greatly reduced by partitioning the chromatogram into regions that are defined by overlaps between the detected peaks. In the simplest case, none of the peaks overlap, and the parameters for each individual peak can be estimated separately.

### Example

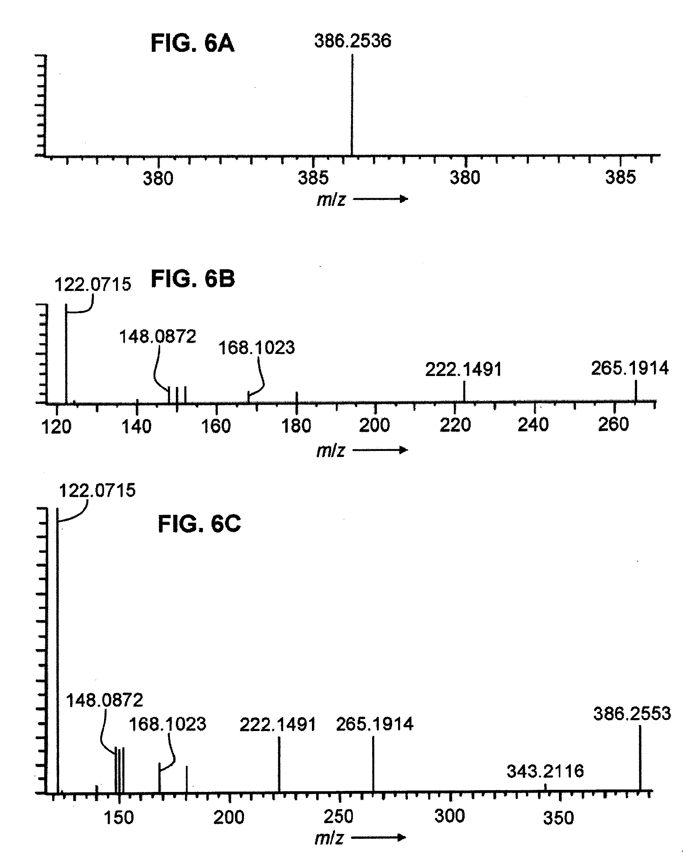

FIG. 6 illustrates an example using tandem mass spectrometry data obtained for the drug buspirone, which is used to treat generalized anxiety disorder (GAD). FIG. 6A is a mass spectrum of a precursor ion generated from buspirone - one of several such precursor ions which could be illustrated. FIG. 6B is a mass spectrum of product ions observed after fragmentation of the buspirone precursor ions. The product-ion peaks illustrated in FIG. 6B comprise only a subset of the full collection of observed product ions, this subset having been generated by filtering the observed product-ion data so as to relate to the illustrated precursor ion peak in accordance with the present teachings. That is, the product ions shown in FIG. 6B are that subset of ions whose masses are consistent with loss of various neutral molecules from the illustrated precursor ion. To test the present methods, the set of filtered data shown in FIG. 6B may be compared with the results (FIG. 6C) of filtering the same raw data using the XIC correlation technique, described elsewhere. Comparison of FIG. 6B with FIG. 6C shows that the two methods produce nearly identical results.

### Conclusion

The end result of methods described in the preceding text and associated figures is a general method to detect peaks and identify matches between precursor ions and product ions generated in all-ions LC/MS/MS analyses without user-adjustable parameters. Since it requires no user input, it is suitable for automation, use in high-throughput screening environments or for use by untrained operators.

The newly invented methods described herein are capable of identifying precursor-ion and product-ion *m*/*z* values in an experiment containing both precursor-ion and product-ion or fragment-ion scans - possibly comprising different data subsets interleaved in time - that are related by neutral loss from a common precursor. Reconstructed MS-2 spectra that are produced by this method very closely reproduce the actual MS-2 spectrum obtained by an instrument capable of selective ion fragmentation. Thus, the novel methods disclosed herein allow multiplexed MS-2 spectral reconstructions from many fragmented ions to be collected and analysed simultaneously. The disclosed novel methods have no user-adjustable parameters, and can be run automatically in a post-acquisition step, or implemented in firmware and the new, simplified output files created at acquisition time. The novel methods disclosed herein may be regarded as complementary to the XIC lineshape correlation methods for reconstructing MS-2 spectra in that the present methods work best in conjunction with high-mass-precision data but do not need well-defined chromatographic lineshapes. This is the reverse of the lineshape correlation methods, which work best with excellent chromatogram lineshape fidelity but which are less dependent on precise mass analysis.

Although the described methods are somewhat computationally intensive, they are nonetheless able to process data faster than it is acquired, and so can be done in real time, so as to make automated real-time decisions about the course of subsequent mass spectral scans on a single sample or during a single chromatographic separation. Such real-time (or near-real-time) decision making processes require data buffering since chromatographic peaks are searched for in a moving window of time - that is, a moving region of interest (ROI). For instruments that generate significant chemical noise, the number of unique ions that are transferred into the output data file can be 1000x fewer than in the original data. The newly invented methods also provide a list of components found, with details presented including but not limited to, chromatographic retention time and peak width, ion mass, and signal to noise characteristics.

Computer instructions according to any of the methods described above may be supplied as a computer program product or products tangibly embodied on any form of computer readable medium, such as disk storage, optical storage or electronic memory device, such computer program product or products and storage devices themselves being aspects of the present teachings.

The discussion included in this application is intended to serve as a basic description. Although the invention has been described in accordance with the various embodiments shown and described, one of ordinary skill in the art will readily recognize that there could be variations to the embodiments and those variations would be within the spirit and scope of the present invention. The reader should be aware that the specific discussion may not explicitly describe all embodiments possible; many alternatives are implicit. Accordingly, many modifications may be made by one of ordinary skill in the art without departing from the spirit, scope and essence of the invention. Neither the description nor the terminology is intended to limit the scope of the invention. Any patents, patent applications, patent application publications or other literature mentioned herein are hereby incorporated by reference herein in their respective entirety as if fully set forth herein except that, insofar as such patents, patent applications, patent application publications or other literature may conflict with the present specification, then the present specification will control.

## Claims

1. A method of acquiring and interpreting data using a mass spectrometer, said method comprising:
(a) generating a multiplexed mass spectrum using the mass spectrometer system, the multiplexed mass spectrum comprising a superposition of a plurality of product-ion mass spectra comprising a plurality of product-ion types having respective product-ion mass-to-charge (*m*/*z*) ratios, each product-ion mass spectrum corresponding to fragmentation of a respective precursor-ion type formed by ionization of a chemical compound, each precursor-ion type having a respective precursor-ion mass-to-charge (*m*/*z*) ratio; and
(b) recognizing a set comprising a precursor-ion type and one or more product-ion types corresponding to each of one or more of the product-ion mass spectra by recognizing one or more losses of a respective valid neutral molecule from each said precursor-ion type.

2. A method as recited in claim 1, wherein the step (a) of generating a multiplexed mass spectrum using the mass spectrometer comprises generating the spectrum with a mass-to-charge precision of 5 parts-per-million or better.

3. A method as recited in claim 1 wherein the step (a) of generating a multiplexed mass spectrum using the mass spectrometer comprises generating the multiplexed mass spectrum as one of a plurality of multiplexed mass spectra generated during sequential introduction into the mass spectrometer system of compounds corresponding to a chromatograph elution peak, wherein the number of multiplexed mass spectra corresponding to the elution peak is fewer than seven.

4. A method as recited in claim 1, wherein the recognizing of one or more losses of a respective valid neutral molecule from each said precursor-ion type comprises:
(b1) determining the charge state and mass of each said precursor-ion type;
(b2) determining the charge state and mass of each of the plurality of product-ion types;
(b3) subtracting the mass of each of the plurality of product-ion types from the mass of each said precursor-ion type so as to generate a list of tentative molecular masses for each pair of precursor-ion type and product-ion type;
(b4) tabulating a list of tentative molecular formulas for each tentative molecular mass;
(b5) ranking each list of tentative molecular formulas according to chemical likelihood rules and an isotopic pattern correspondence;
(b6) assigning the highest-ranked tentative molecular formula to its respective tentative molecular mass if the ranking of the highest-ranked tentative molecular formula exceeds a threshold value; and
(b7) for each pair of precursor-ion type and product-ion type corresponding to a tentative molecular mass corresponding to an assigned tentative molecular formula, recognizing the assigned tentative molecular formula as a loss of a valid neutral molecule.

5. A method as recited in claim 4, wherein the step (b5) of ranking each list of tentative molecular formulas according to chemical likelihood rules and an isotopic pattern correspondence comprises:
(b5a) calculating a plurality of likelihood scores for each tentative molecular formula, each likelihood score corresponding to a respective rule; and
(b5b) calculating the ranking for each tentative molecular formula as the product of the plurality of the likelihood scores multiplied by an isotopic pattern correlation score.

6. A method as recited in claim 4, wherein the step (b5) of ranking each list of tentative molecular formulas according to chemical likelihood rules and an isotopic pattern correspondence comprises:
(b5a) positively excluding a subset of the tentative molecular formulas based on the chemical likelihood rules; and
(b5b) calculating the ranking for each non-excluded tentative molecular formula as an isotopic pattern correlation score.

7. An apparatus comprising:
(i) a mass spectrometer system; and
(ii) a programmable electronic processor electrically coupled to the mass spectrometer, the programmable processor comprising instructions operable to cause the programmable processor to:
(a) cause the mass spectrometer system to generate a multiplexed mass spectrum using the mass spectrometer system, the multiplexed mass spectrum comprising a superposition of a plurality of product-ion mass spectra comprising a plurality of product-ion types having respective product-ion mass-to-charge (*m*/*z*) ratios, each product-ion mass spectrum corresponding to fragmentation of a respective precursor-ion type formed by ionization of a chemical compound, each precursor-ion type having a respective precursor-ion mass-to-charge (*m*/*z*) ratio;
(b) receive the multiplexed mass spectrum from the mass spectrometer system; and
(c) recognize a set comprising a precursor-ion type and one or more product-ion types corresponding to each of one or more of the product-ion mass spectra by recognizing one or more losses of a respective valid neutral molecule from each said precursor-ion type.

8. An apparatus as recited in claim 7, further comprising:
(iii) a chromatograph for providing a stream of separated chemical substances to the mass spectrometer system,
wherein the multiplexed mass spectrum comprises one of a plurality of multiplexed mass spectra generated during sequential introduction into the mass spectrometer system of the separated chemical substances corresponding to a chromatograph elution peak, wherein the number of multiplexed mass spectra corresponding to the elution peak is fewer than seven.

9. An apparatus as recited in claim 7, wherein the mass spectrometer system includes a time-of-flight (TOF) mass analyzer.

10. An apparatus as recited in claim 7, wherein the mass spectrometer system includes an electrostatic trap mass analyzer.
